# EUROPÄISCHE PATENTSCHRIFT

(11) **EP 1 386 588 B1**
(45) Veröffentlichungstag und Bekanntmachung des Hinweises auf die Patenterteilung: **17.05.2006**
(21) Anmeldenummer: 03011806.1
(22) Anmeldetag: 26.05.2003
(51) Int. Cl.: A61B 17/17

(54) **Zielgerät für einen Verriegelungsnagel**
Aiming guide for use with intramedullary nail
Guide de visée pour clou intramédullaire

(30) Priorität: 01.08.2002 DE 20211806 U
(43) Veröffentlichungstag der Anmeldung: 04.02.2004
(73) Patentinhaber: Stryker Trauma GmbH, 24232 Schönkirchen (DE)
(72) Erfinder: Zander, Nils, 24340 Eckernförde (DE)
(74) Vertreter: Hauck, Graalfs, Wehnert, Döring, Siemons

(56) Entgegenhaltungen:
- EP-A- 0 948 936
- US-A- 5 334 192
- US-A1- 2002 058 949
- PATENT ABSTRACTS OF JAPAN vol. 2002, no. 02, 2. April 2002 (2002-04-02) & JP 2001 286480 A (HOMUZU GIKEN:KK), 16. Oktober 2001 (2001-10-16)

## Beschreibung

Die Erfindung bezieht sich auf ein Zielgerät für einen Verriegelungsnagel nach dem Oberbegriff des Patentanspruchs 1.

In den Unterschenkel- oder Oberschenkelknochen einführbare Verriegelungsnägel weisen zumeist mehrere Querbohrungen auf, durch die hindurch Knochenschrauben geführt werden, um die Verriegelungsnägel sicher im Knochenkanal zu halten. Ein besonderes Problem bei Verriegelungsnägeln ist das Auffinden der Querbohrungen beim implantierten Nagel. Hierfür werden Zielgeräte verwendet. Eine Kategorie von Zielgeräten arbeitet mit Röntgenstrahlen, und die Querbohrungen des Verriegelungsnagels in dem Knochen werden auf einem Monitor abgebildet. Ferner findet eine Abbildung eines Zielelements statt. Auf diese Weise ist es möglich, an der Außenseite des Knochens die Stelle zu markieren, die auf der Achse der Querbohrungen liegt.

Bei einer anderen Kategorie sind die Zielgeräte fest mit dem implantierten Nagel verbunden. Ein bügelartiger Abschnitt weist mindestens eine Durchbohrung auf, deren Achse mit der Achse einer Querbohrung des Nagels ausgerichtet ist, wenn er am Zielgerät angebracht ist. Zur Führung des Bohrwerkzeuges bzw. der Knochenschraube ist auch bekannt, durch die Querbohrung des Zielgeräts eine Führungshülse zu stecken, die bis gegen die Außenseite des Knochen vorgeschoben wird.

Die Führungshülse sitzt passend in der Querbohrung des Zielgeräts, muß jedoch von Hand relativ leicht bewegt werden können, um dem Chirurgen die Arbeit nicht zu erschweren. Dadurch besteht die Gefahr, daß bei der Handhabung des Zielgerätes bzw. beim Bohren und auch beim Einschrauben von Knochenschrauben die Führungshülse nach hinten verrutscht. Aus EP 0 948 936 A (Basis für den Oberbegriff des Anspruchs 1) ist bekannt geworden, an dem Aufnahmeabschnitt, der die Querbohrung oder Zielbohrung des Zielgeräts aufweist, einen federnden Hebel anzuformen, der eine mit der Querbohrung ausgerichtete Bohrung aufweist. Bei entspanntem federndem Hebel ist die Hülse leicht klemmend gehalten und wird freigegeben, wenn der federnde Hebel in Richtung des Aufnahmeabschnitts verformt wird.

Zur Versorgung von Brüchen im Femurkopf- und -halsbereich ist bekannt, zwei längere Knochenschrauben annähernd parallel einzusetzen, die von entsprechenden schrägen Querbohrungen eines Verriegelungsnagels gehalten sind. Der Aufnahmeabschnitt des Zielgeräts ist mit entsprechenden schrägen Zielbohrungen versehen. Um auch für diesen Versorgungsfall beide Führungshülsen klemmend, jedoch lösbar zu halten, ist denkbar, den federnden Abschnitt des bekannten Zielgeräts mit zwei Querdurchgängen zu versehen. Nachteilig ist hierbei aber, daß stets nur beide Hülsen zugleich geklemmt oder freigegeben werden können.

Der Erfindung liegt die Aufgabe zugrunde, ein Zielgerät für einen Verriegelungsnagel zu schaffen, bei dem zwei Führungshülsen auf einfache Weise verstellt und in der jeweiligen Position fixiert werden können. Insbesondere soll eine Verstellbarkeit und Positionierung von zwei parallelen Führungshülsen ermöglicht werden.

Diese Aufgabe wird durch die Merkmale des Patentanspruchs 1 gelöst.

Bei dem erfindungsgemäßen Zielgerät weist der Aufnahmeabschnitt einen länglichen Schlitz auf, der sich in Längsrichtung des Aufnahmeabschnitts erstreckt, wobei diese Erstreckung quer zur Durchbohrung im Aufnahmeabschnitt ist. In dem Schlitz ist der Hebel aus elastisch nachgebendem Material angebracht, indem ein Endbereich des Hebels an einem Ende des Schlitzes befestigt wird. Der andere End-bereich des Hebels weist eine aus dem Schlitz heraus stehende Handhabe auf. Schlitz und Hebel sind so angeordnet, daß der Hebel bei Betätigung der Handhabe in einer Ebene verschwenkt wird, die senkrecht auf der Querbohrung des Aufnahmeabschnitts steht. Im Hebel ist eine Ausnehmung oder eine Öffnung geformt, durch welche sich die Führungshülse hindurch erstrecken kann, wobei jedoch eine aufgenommene Führungshülse klemmend gehalten ist, indem die Ausnehmung oder Öffnung des Hebels etwas außermittig liegt, wenn der Hebel im entspannten Zustand ist. Wird der Hebel hingegen leicht verschwenkt, stellt sich eine Ausrichtung der Ausnehmung oder Öffnungen mit der Querdurchbohrung ein, und die Führungshülse läßt sich leicht in die gewünschten Richtung verschieben.

Besonders vorteilhaft ist die erfindungsgemäße Lösung bei ihrer Anwendung auf zwei schräge, relativ eng zusammenliegende Querbohrungen im Aufnahmeabschnitt bei der Implantierung eines Verriegelungsnagels als Träger für zwei parallele Knochenschrauben zur Versorgung von Brüchen im Femurhals- und -kopfbereich. Zu diesem Zweck weist der Aufnahmeabschnitt zwei eng zusammenliegende schräge Durchbohrungen auf. Der Aufnahmeabschnitt weist zwei parallele Schlitze auf, von denen jeder einen Hebel aufnimmt. Jeder Hebel weist zwei benachbarte Öffnungen oder Ausnehmungen auf und die Öffnungen oder Ausnehmungen sind so ausgebildet, daß parallele Hülsen einzeln und getrennt unabhängig voneinander klemmend gehalten und lösbar sind. Die Betätigung eines Hebels zur Verschiebung einer Führungshülse führt mithin nicht zum Lösen der anderem Hülse, vielmehr bleibt diese in ihrer Position stehen. Die Erfindung ermöglicht außerdem ein außerordentlich enges Zusammenbringen der beiden Knochenschrauben. Bekanntlich gibt der Durchmesser des Femurhalses den maximalen Abstand der beiden einzuführenden Knochenschrauben vor.

Der Hebel kann so geformt sein, daß er in sich elastisch verformbar ist, vergleichbar einem einendig eingespannten Biegebalken. Nach einer Ausgestaltung der Erfindung ist vorgesehen, daß ein Endabschnitt des Hebels passend in einem Befestigungsabschnitt des Schlitzes aufgenommen und befestigt ist, beispielsweise durch Klebung. Der Endabschnitt ist über einen Verbindungsabschnitt von relativ kleinem Querschnitt mit dem ersten Teil des Hebels verbunden. Somit findet die Verformung bei Betätigung des Hebels im wesentlichen im Verbindungsabschnitt statt. Eine Verformung der Ausnehmung oder Öffnung bei Betätigung des Hebels findet dabei nicht statt.

Nach einer weiteren Ausgestaltung der Erfindung ist der Aufnahmeabschnitt an dem Zielarm des Zielgerätes verschiebbar, jedoch feststellbar gelagert. Nach einer Ausgestaltung der Erfindung erstreckt sich der Zielarm im spitzen Winkel zur Achse, auf der sich der am Zielarm befestigte Verriegelungsnagel befindet, und der Aufnahmeabschnitt weist einen im stumpfen Winkel abgewinkelten Anschlußabschnitt auf, der verschiebbar auf dem Zielarm geführt ist. Zielarm und der Anschlußabschnitt sind so ausgebildet, daß der Aufnahmeabschnitt annähernd parallel zur Nagelachse verläuft.

Aufnahmeabschnitt und/oder Hebel sind vorzugsweise aus einem mit Kohlefasern verstärkten Kunststoff geformt. Dieses Material weist eine ausreichende Festigkeit für ein Zielgerät auf, hat jedoch den Vorteil, für Röntgenstrahlung transparent zu sein.

Ein Ausführungsbeispiel der Erfindung soll nachfolgend anhand von Zeichnungen näher erläutert werden.
- Fig. 1: zeigt perspektivisch ein Zielgerät nach der Erfindung mit aufgenommenem Verriegelungsnagel.
- Fig. 2: zeigt perspektivisch den Aufnahmeabschnitt des Zielgeräts nach Fig. 1.
- Fig. 3: zeigt die Seitenansicht des Aufnahmeabschnitts des Zielgeräts nach Fig. 1 in Richtung Pfeil 3.
- Fig. 4: zeigt einen Schnitt durch die Darstellung nach Fig. 3 entlang der Linie 4-4.
- Fig. 5: zeigt eine weitere perspektivische Ansicht des Aufnahmeabschnitts des Zielgeräts nach Fig. 1 ohne aufgenommene Hebel.
- Fig. 6: zeigt einen Schnitt durch einen Teil des Zielarms des Zielgeräts nach Fig. 1 im Bereich der Verbindungsstelle mit dem Verriegelungsnagel.
- Fig. 7: zeigt den Hebel nach Fig. 2 im rechten Schlitz des Aufnahmeabschnitts nach Fig. 2.
- Fig. 8: zeigt den Hebel im linken Aufnahmeschlitz des Aufnahmeabschnitts nach Fig. 2.

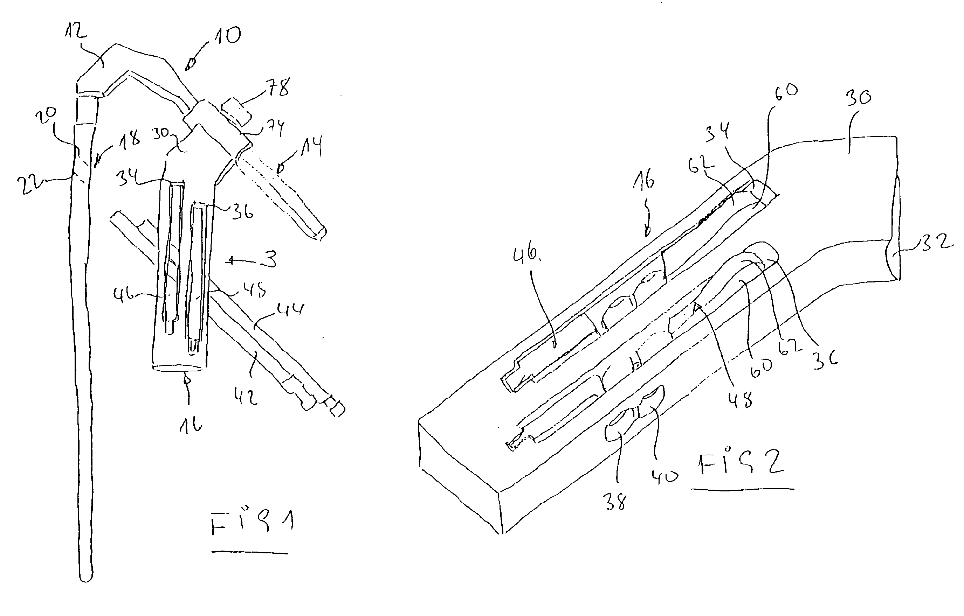

In Figur 1 ist ein Zielgerät 10 zu erkennen, das einen ersten Abschnitt 12 aufweist, an dem ein Zielarm 14 einteilig angebracht ist. Auf dem Zielarm verschiebbar ist ein Aufnahmeabschnitt 16 angeordnet. Am ersten Abschnitt 12 ist ein Verriegelungsnagel 18 befestigt. Der Verriegelungsnagel 18 weist zwei schräge Querbohrungen auf, welche durch ihre Achsen 20 und 22 angedeutet sind. Der Verriegelungsnagel 18 weist auch im distalen Bereich die Querbohrungen auf, die jedoch nicht gezeigt sind.

Der Verriegelungsnagel 18 dient zur Aufnahme von zwei parallelen eng zusammenliegenden Knochenschrauben zur Versorgung einer Fraktur im Bereich des Femurkopfes oder -halses.

Wie aus Figur 6 erkennbar, weist der Abschnitt 12 eine Aufnahmebohrung 24 auf für eine Hülse 26, die mit dem Ende des Verriegelungsnagels 18 zusammenwirkt. Die Hülse hat am unteren freien Ende einen achsparallelen Vorsprung 28, der mit einer nicht gezeigten Ausnehmung des Verriegelungsnagels 18 zusammenwirkt, um die Drehlage des Verriegelungsnagels zu bestimmen. Durch die Aufnamebohrung 24 und die Hülse 26 wird ein Befestigungsbolzen hindurchgeführt zum Festschrauben des Nagels 18 am Zielgerät 10 (im einzelnen nicht gezeigt). Eine Gewindebohrung 23 dient für die Anbringung eines Anschlags oder dergleichen, über den das Zielgerät und damit der Verriegelungsnagel 18 mittels eines Schlaginstruments betätigt werden kann.

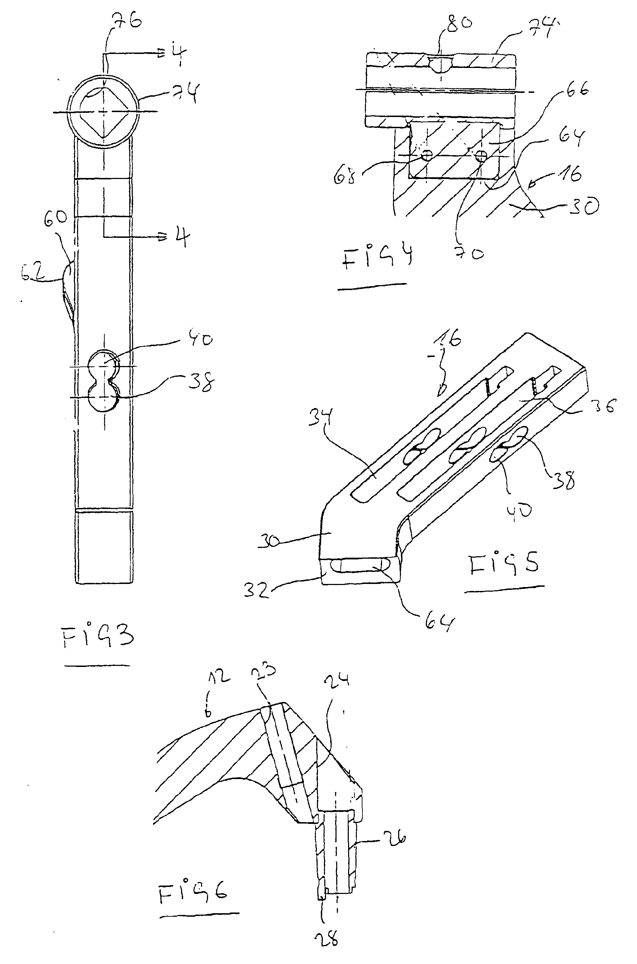

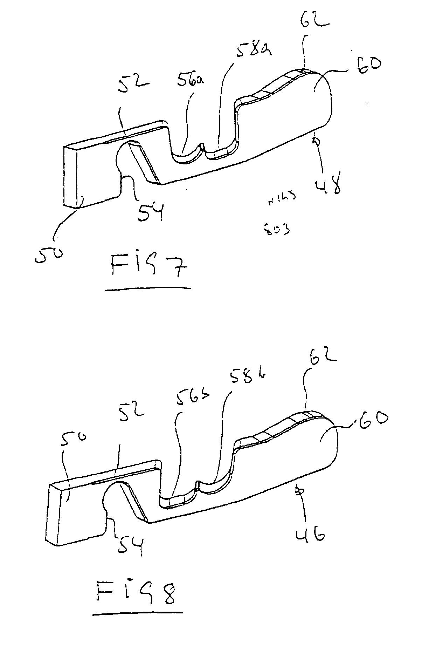

Der Aufbau des Aufnahmeabschnitts 16 geht näher aus den Figuren 2, 3, 4 und 5 hervor. Man erkennt, daß der Aufnahmeabschnitt 16 einen im Querschnitt rechteckigen länglichen Körper darstellt, der an einem Ende einen im stumpfen Winkel abgewinkelten Abschnitt 30 besitzt, der am freien Ende eine im Schnitt kreisbogenförmige Hohlkehle 32 aufweist. Der Aufnahmeabschnitt 16 weist zwei parallele Schlitze 34, 36 auf, die vollständige durch den Aufnahmeabschnitt 16 hindurchgehen. Wie aus der Figur 1 ersichtlich, erstrecken sich die Schlitze 34, 36 in eine Ebene, die parallel zur Achse des Verriegelungsnagels 18 liegt. Der Aufnahmeabschnitt 16 erstreckt sich ebenfalls parallel zur Nagelachse.

Durch die einzelnen Stege des Aufnahmeabschnitts, welche durch die Schlitze 34, 36 gebildet sind, erstrecken sich zwei parallele schräge Durchbohrungen 38, 40. Sie liegen so nahe zusammen, daß die Wandung der Durchbohrungen 38, 40 im angrenzenden Bereich offen ist. Die Durchbohrungen 38, 40 dienen zur Aufnahme von Führungshülsen 42, 44 (Fig. 1), welche auf diese Weise außerordentlich nahe zusammengebracht werden können. Die Führungshülsen haben die an sich bekannte Funktion, Werkzeuge beim Einsetzen von Knochennägeln bzw. Knochennägel zu führen bei deren Implantierung, nachdem zuvor in an sich bekannter Weise in der Kortikalis des Knochens Bohrungen vorgenommen worden sind. Im übrigen wird auf die Beschreibung eines Zielgeräts vergleichbaren Aufbaus gemäß EP 0 948 936 verwiesen.

In den Schlitzen 34, 36 sind Hebel 46, 48 angeordnet. Der Aufbau der Hebel 46, 48 geht aus den Figuren 7 und 8 hervor. Die Hebel sind annähernd gleich aufgebaut. Daher sollen gleiche Teile mit gleichen Bezugszeichen versehen werden. Die Hebel 46, 48 weisen einen ersten Endabschnitt 50 auf, der, wie in Figur 2 zu erkennen, in einem schmaleren Endbereich der Schlitze 34, 36 angeordnet und darin durch Klebung befestigt ist. Eine zusätzliche Befestigung kann mit Hilfe von nicht gezeigten Stiften erfolgen. An den Endabschnitt 50 schließt sich ein oberer Verbindungsabschnitt 52 von relativ geringem Querschnitt an, der dadurch gebildet ist, daß eine Querausnehmung 54 im Hebel 46, 48 geformt ist. Im mittleren Bereich der Hebel 46, 48 befinden sich Ausnehmungen 56a, 58a bzw. 56b bzw. 58b. Nach rechts schließt sich eine Handhabe 60 an, die eine etwas nach oben ragende Wölbung 62 aufweist. Im eingebauten Zustand erstreckt sich die Wölbung 62 etwas über die zugeordnete Fläche des Aufnahmeabschnitts 16 hinaus (siehe Fig. 2 und 3). Die Ausnehmungen 56a, 58a und 56b, 58b sind nun so geformt, daß eine Führungshülse, welche durch die Querbohrung 38 hindurchgeführt ist, klemmend mit dem Hebel 48 zusammenwirkt, wenn dieser im entspannten Zustand ist. Eine Hülse, welche durch die Querbohrung 40 hindurchgeführt ist, wird durch Ausnehmung 58b des Hebels 46 festgeklemmt, wenn dieser Hebel im entspannten Zustand ist. Mithin wird jede Hülse jeweils von einem Hebel klemmend gehalten, wenn die Hebel im entspannten Zustand sind. Wird einer der Hebel 46, 48 durch Drücken auf die Wölbung 62 in Richtung Schlitz leicht verformt, wird die von diesem Hebel geklemmte Hülse freigegeben, so daß diese verschoben werden kann. Mithin können unabhängig voneinander die Hülsen 42, 44 verschoben und in ihrer Lage gehalten werden.

Wie sich aus Figur 5 ergibt, hat der abgewinkelte Anschlußabschnitt 30 am freien Ende eine Ausnehmung 64. Sie dient zur Aufnahme eines Verbindungsstücks 66 in Form eines Stahlplättchens, das in der Ausnehmung 64 mittels zweier Stifte 68, 70 gehalten und möglicherweise zusätzlich durch Klebung befestigt ist. Das Stahlplättchen 66 steht etwas über den Boden der Hohlkehle 32 über. Eine Führungshülse 74 aus Stahl hat einen Durchgang mit einem unrunden Querschnitt, wie bei 76 zu erkennen. Das Querschnittsprofil des Zielarms 14 ist komplementär zum Querschnitt des Durchgangs der Führungshülse 74. Die Führungshülse 74, die einen kreisförmigen Außenumfang hat, ist passend in die Kehle 32 eingesetzt und dort verklebt. Eine äußere Ausnehmung der Führungshülse 74 wirkt mit dem überstehenden Teil des Stahlplättchens 66 zusammen, wodurch die Führungshülse zusätzlich wirksam am Aufnahmeabschnitt 16 gehalten ist. Mit Hilfe der Führungshülse kann mithin der Aufnahmeabschnitt entlang des Zielarms 14 bewegt werden, wobei jedoch eine Drehung verhindert wird.

Eine Feststellschraube 78 ist in ein Loch 80 der Führungshülse 74 eingeschraubt und dient zur Festsetzung des Aufnahmeabschnitts 16 auf dem Zielarm 14.

Der Aufnahmeabschnitt 16 ist aus kohlenstofffaserverstärktem Kunststoff geformt, ebenso wie die Hebel 46, 48. Sie sind mithin für Röntgenstrahlung durchlässig. Die übrigen Teile sind aus korrosionsfreiem Stahl.

## Patentansprüche

1. Zielgerät für ein Verriegelungsnagel mit einem ersten Abschnitt (12), der mit dem zugeordneten Ende des Verriegelungsnagels (18) lösbar verbindbar ist, einem mit dem ersten Abschnitt verbundenem Zielarm (14), der mit einem Aufnahmeabschnitt (16) verbunden ist, der annähernd parallel zum Verriegelungsnagel (18) verläuft, wenn der Verriegelungsnagel (18) mit dem ersten Abschnitt (12) verbunden ist und der mindestens eine Querbohrung (38, 40) aufweist, für die annähernd passende Aufnahme einer Führungshülse (42, 44), wobei der Aufnahmeabschnitt (16) einen Hebel aus elastischem Material aufweist, der relativ zum Aufnahmeabschnitt (16) gegen Federkraft bewegbar ist, wobei die Querbohrung (38, 40) und der Hebel (46, 48) so angeordnet sind, daß bei entspanntem Hebel (46, 48) eine in der Querbohrung (38, 40) aufgenommene Führungshülse (42, 44) leicht klemmend vom Hebel (46, 48) gehalten ist und bei einer gewissen Verstellung des Hebels (46, 48) in eine vorgegebene Richtung frei verschiebbar ist, **dadurch gekennzeichnet, daß** der Aufnahmeabschnitt (16) einen länglichen Schlitz (34, 36) aufweist, der den Hebel (46, 48) aus elastisch nachgebendem Material aufnimmt und der mit einem Endbereich an einem Ende des Schlitzes (34, 36) befestigt ist und im anderen Endbereich eine aus dem Schlitz heraustretende Handhabe (60, 62) aufweist, wobei der Schlitz (34, 36) und der Hebel (46, 48) so angeordnet sind, daß der Hebel (46, 48) bei Betätigung der Handhabe (60, 62) in einer Ebene verschwenkt wird, die senkrecht auf der Achse der Querbohrung (38, 40) steht und im Hebel (46, 48) eine Ausnehmung (56a, 58a, 56b, 58b) oder Öffnung geformt ist, durch welche sich die in der Querbohrung (38, 40) aufgenommene Hülse (42, 44) hindurch erstreckt.

2. Zielgerät nach Anspruch 1, **dadurch gekennzeichnet, daß** der Aufnahmeabschnitt (16) zwei eng zusammenliegende schräge Querbohrungen (38, 40) aufweist, der Aufnahmeabschnitt (16) zwei parallele Schlitze (34, 36) aufweist, von denen jeder einen Hebel (46, 48) aufnimmt, jeder Hebel zwei benachbarte Öffnungen oder Ausnehmungen (56a, 58a, 56b, 58b) aufweist und die Öffnungen oder Ausnehmungen so ausgebildet sind, daß parallele Hülsen (42, 44) einzeln und getrennt unabhängig voneinander klemmend gehalten und lösbar sind.

3. Zielgerät nach Anspruch 1 oder 2, **dadurch gekennzeichnet, daß** ein Endabschnitt (50) des Hebels (46, 38) passend in einem Befestigungsabschnitt des Schlitzes (46, 48) aufgenommen und befestigt ist und der Endabschnitt (50) über einen Verbindungsabschnitt (52) von relativ kleinem Querschnitt mit dem ersten Teil des Hebel (46, 48) verbunden ist.

4. Zielgerät nach Anspruch 3, **dadurch gekennzeichnet, daß** der Endabschnitt des Hebels (46, 48) im Schlitz (34, 36) durch Klebung befestigt ist.

5. Zielgerät nach einem der Ansprüche 1 bis 4, **dadurch gekennzeichnet, daß** der Aufnahmeabschnitt (16) an dem Zielarm (14) verschiebbar, jedoch feststellbar gelagert ist.

6. Zielgerät nach Anspruch 5, **dadurch gekennzeichnet, daß** der Aufnahmeabschnitt (16) einen mit stumpfem Winkel abgewinkelten Anschlußabschnitt (30) aufweist und der Zielarm (14) im spitzen Winkel zur Achse des aufgenommenen Nagels (18) verläuft derart, daß der Aufnahmeabschnitt (16) sich annähernd parallel zur Nagelachse erstreckt.

7. Zielgerät nach einem der Ansprüche 1 bis 6, **dadurch gekennzeichnet, daß** der Aufnahmeabschnitt (16) und/oder die Hebel (46, 48) aus kohlefaserverstärktem Kunststoff geformt sind.

8. Zielgerät nach den Ansprüche 4 und 6, **dadurch gekennzeichnet, daß** mit dem Aufnahmeabschnitt (16) eine Führungshülse (74) verbunden ist, die unverdrehbar auf dem Zielarm (14) geführt ist.

## Claims

1. Aiming device for a locking nail with a first portion (12), which is detachably connectable with the associated end of the locking nail (18), an aiming arm (14) connected with the first portion (12), said aiming arm being connected with an accommodation portion (16) which runs approximately parallel to the locking nail (18) when the locking nail (18) is connected with the first portion (12) and which is provided with at least one transversal bore (38, 40) for the approximately fitting accommodation of a guiding sleeve (42, 44), the accommodation portion (16) being provided with a lever made of an elastic material, which is movable in relation to the accommodation portion (16) against a spring force, and the transversal bore (38, 40) and the lever (46, 48) being disposed such that upon relaxed lever (46, 48) guiding sleeve (42, 44) accommodated in the transversal bore (38, 40) is held slightly clamped by the lever (46, 48) and is freely movable in a predetermined direction upon a certain adjustment of the lever (46, 48), **characterised in that** the accommodation portion (16) is provided with an oblong slit (34, 36), receiving the lever (46, 48) of elastically yielding material, said lever being attached to one end of the slit (34, 36) with one end region and is provided on the other end region with a handle (60, 62) sticking out from the slit, the slit (34, 36) and the lever (46, 48) being disposed such that that the lever (46, 48), upon operation of the handle (60, 62), is swivelled in a plane which is vertical to the axis of the transversal bore (38, 40), and a recess (56a, 58a, 56b, 58b) or opening is formed in the lever (46, 48), through which the sleeve (42, 44) accommodated in the transversal bore (38, 40) extends through.

2. Aiming device according to claim 1, **characterised in that** the accommodation portion (16) is provided with two skew bores (38, 40) closely spaced together, the accommodation portion (16) is provided with two parallel slits (34, 36), of which each one accommodates one lever (46, 48), each lever is provided with two adjacent openings or recesses (56a, 58a, 56b, 58b), and the openings or recesses are formed such that parallel sleeves (42, 44) are individually and separately, independent from each other, held clamping and are detachable.

3. Aiming device according to claim 1 or 2, **characterised in that** an end portion (50) of the lever (46, 38) is fittingly accommodated and fixed in a fixing portion of the slit (46, 48) and the end portion (50) is connected with the first part of the lever (46, 48) via a connecting portion (52) of relatively small cross section.

4. Aiming device according to claim 3, **characterised in that** the end portion of the lever (46, 48) is fixed in the slit (34, 36) by glueing.

5. Aiming device according to one of claims 1 to 4, **characterised in that** the accommodation portion (16) is movably, but fixably mounted on an aiming arm (14).

6. Aiming device according to claim 5, **characterised in that** the accommodation portion (16) is provided with an annexation portion (30) offset with an obtuse angle, and the aiming arm (14) runs in an acute angle with respect to the axis of the accommodated nail (18), such that the accommodation portion (16) extends approximately parallel to the nail axis.

7. Aiming device according to one of claims 1 to 6, **characterised in that** the accommodation portion (16) and/or the levers (46, 48) are formed from carbon fiber reinforced plastic material.

8. Aiming device according to the claims 4 and 6, **characterised in that** with the accommodation portion (16) is connected a sleeve portion (74), which is guided in a not rotatable manner on the aiming arm (14).

## Revendications

1. Appareil de visée pour un clou de verrouillage, comportant une première partie (12), qui est apte à être assemblée de manière amovible avec l'extrémité correspondante du clou de verrouillage (18), un bras de visée (14), qui est relié à la première partie (12), qui est à peu près parallèle au clou de verrouillage (18), lorsque le clou de verrouillage (18) est assemblé à la première partie (12), et qui comporte au moins un trou transversal (38, 40) destiné à recevoir de manière sensiblement ajustée un manchon de guidage (42, 44), la partie de réception (16) comportant un levier en matériau élastique, qui peut être déplacé par rapport à la partie de réception (16) dans le sens opposé à une force de ressort, le trou transversal (38, 40) et le levier (46, 48) étant agencés de telle sorte que, lorsque le levier (46, 48) est à l'état détendu, un manchon de guidage (42, 44), reçu dans le trou transversal (38, 40), est maintenu avec un léger serrage par le levier (46, 48) et, en cas d'un certain déplacement du levier (46, 48), peut être déplacé librement dans une direction prédéfinie, **caractérisé en ce que** la partie de réception (16) comporte une fente longitudinale (34, 36), qui reçoit le levier (46, 48), qui est réalisé dans un matériau élastiquement flexible et qui est fixé avec une zone d'extrémité contre une extrémité de la fente (34, 36) et comporte dans l'autre zone d'extrémité une manette (60, 62) sortant de la fente, la fente (34, 36) et le levier (46, 48) étant agencés de telle sorte que le levier (46, 48), en cas de manipulation de la manette (60, 62), pivote dans un plan qui est perpendiculaire à l'axe du trou transversal (38, 40), et un évidement (56a, 58a, 56b, 58b) ou ouverture étant réalisé dans le levier (46, 48), à travers lequel passe le manchon (42, 44) reçu dans le trou transversal (38, 40).

2. Appareil de visée selon la revendication 1, **caractérisé en ce que** la partie de réception (16) comporte deux trous transversaux (38, 40) obliques, disposés étroitement l'un contre l'autre, **en ce que** la partie de réception (16) comporte deux fentes (34, 36) parallèles, dont chacune reçoit un levier (46, 48), **en ce que** chaque levier comporte deux ouvertures ou évidements (56a, 58a, 56b, 58b) adjacents et **en ce que** les ouvertures ou évidements sont réalisés de telle sorte que des manchons (42, 44) parallèles peuvent être maintenus par serrage et sont amovibles individuellement et séparément, indépendamment l'un de l'autre.

3. Appareil de visée selon la revendication 1 ou 2, **caractérisé en ce qu'**une partie d'extrémité (50) du levier (46, 48) est reçue et fixée de manière ajustée dans un tronçon de fixation de la fente (34, 36) et **en ce que** la partie d'extrémité (50) est reliée à la première partie du levier (46, 48) par l'intermédiaire d'une partie de liaison (52) avec une section relativement petite.

4. Appareil de visée selon la revendication 3, **caractérisé en ce que** la partie d'extrémité du levier (46, 48) est fixée par collage dans la fente (34, 36).

5. Appareil de visée selon l'une quelconque des revendications 1 à 4, **caractérisé en ce que** la partie de réception (16) est montée coulissante sur le bras de visée (14), et néanmoins de manière à pouvoir être immobilisée.

6. Appareil de visée selon la revendication 5, **caractérisé en ce que** la partie de réception (16) comporte une partie d'assemblage (30) coudée selon un angle obtus et **en ce que** le bras de visée (14) s'étend en formant un angle aigu avec l'axe du clou (18) reçu, de telle sorte que la partie de réception (16) est sensiblement parallèle à l'axe du clou.

7. Appareil de visée selon l'une quelconque des revendications 1 à 6, **caractérisé en ce que** la partie de réception (16) et/ou le levier (46, 48) sont réalisés dans une matière plastique renforcée par des fibres de carbone.

8. Appareil de visée selon les revendications 4 et 6, **caractérisé en ce qu'**un manchon de guidage (74), qui est monté de manière fixe en rotation sur le bras de visée (14), est relié à la partie de réception (16).
